# EUROPEAN PATENT SPECIFICATION

(11) **EP 3 035 941 B1**
(45) Date of publication and mention of the grant of the patent: **03.10.2018**
(21) Application number: 14766010.4
(22) Date of filing: 13.08.2014
(51) Int. Cl.: A61K 31/7088, C12N 15/113

(54) **USE OF A MIR172 MOLECULE FOR DECREASING INFLAMMATION**
VERWENDUNG EINES MIR172-MOLEKÜLS ZUR LINDERUNG VON ENTZÜNDUNGEN
UTILISATION D'UNE MOLÉCULE MIR172 POUR DIMINUER L'INFLAMMATION

(30) Priority: 23.08.2013 PL 40512513
(43) Date of publication of application: 29.06.2016
(73) Proprietor: Instytut Biochemii i Biofizyki PAN, 02-106 Warszawa (PL); Uniwersytet Im. Adama Mickiewicza W Poznaniu, 61-712 Poznan (PL)
(72) Inventor: LUKASIK, Anna, 62-510 Konin (PL); ZIELENKIEWICZ, Piotr, 00-248 Warszawa (PL); SZWEYKOWSKA-KULINSKA, Zofia, 61-717 Poznan (PL); PACZEK, Leszek, 03-610 Warszawa (PL); NOWACZYK, Maria, 02-759 Warszawa (PL)
(74) Representative: Twardowska, Aleksandra
(86) International application number: PCT/PL2014/000092
(87) International publication number: WO 2015/026249

(56) References cited:
- WO-A1-2008/116267
- WO-A1-2010/118979
- WO-A2-2005/035769
- WO-A2-2008/142567
- WO-A2-2010/151755

## Description

Described is the use of plant-derived miR172 molecule or its synthetic equivalent, selected from amongst miR172a or miR172b, for decreasing inflammatory processes in the organism, a method of decreasing B and T lymphocyte proliferation, as well as a method of reducing protein FAN (Factor Associated with Neutral Sphingomyelinase Activation) level. The goal of the present invention is to deliver a novel therapeutic method based on miRNA molecules, which through the interaction with mRNA encoding FAN protein, negatively regulate its expression and decrease the inflammatory response of the organism.

MicroRNAs (miRNAs) are a class of single-stranded, regulatory RNAs that are widely evolutionarily conserved among many known species [1-3]. These short (18-24 nt), non-coding molecules mediate post-transcriptional gene expression regulation by promoting cleavage or inhibiting translation of the target mRNA [4-6]. As a mature sequence forms, miRNAs are generated in multi-step process, which begins with miRNA gene transcription into long primary transcript with many stem-loop units (pri-miRNA). The pri-miRNA is further processed into the hairpin precursor (pre-miRNA) and cleaved to generate miRNA:miRNA* duplex with two nucleotide overhangs at the 3' end. Described, individual stages of miRNAs precursors processing are slightly different in animal and plant organisms [7-10]; however, the final step of miRNAs maturation process is similar in both cases. Namely, one of the duplex strands (*-strand) is usually degraded; whereas the second strand is loaded on the RISC (RNA-Induced Silencing Complex) multicomplex, which binds to the specific mRNA transcript [9-13]. Through this hybridization, miRNAs negatively regulate the expression of target genes thereby controlling cell development, apoptosis, proliferation, differentiation and other important functions in living organisms [9, 14, 15]. In humans, several reports have associated the expression profiles of specific miRNAs with certain physiological and pathological stages, tumorigenesis or even patient response to treatment. Thus, in medicine miRNAs become new diagnostic and prognostic biomarkers [16, 17], and have been incorporated in a few novel therapies for treating several human disorders [18, 19]. In the medical context, miRNAs are also well known for their involvement in the regulation of organism immune response, immunity or inflammation. miRNAs' effect on these processes is connected among others with the: (1) interferon and cytokines signalling pathways, (2) development and differentiation regulation of B and T lymphocytes, macrophages, monocytes, NK cells, as well as granulocytes, or (3) activation of some of these cells, which are part of innate and adaptive immune system [20-23]. The aforementioned T and B lymphocytes and factors such as the tumor necrosis factor alpha (TNF-a) play an important, indeed: critical, role in a number of autoimmune diseases - e.g., rheumatoid arthritis, psoriasis, atherosclerosis, Crohn's disease and multiple sclerosis [24-31]. At present, there are several known methods; of treating autoimmune diseases, such as anti-TNF-α therapy; however, due to their significant adverse effects (including increase susceptibility to infections) further research are carried out to find novel therapies, which will allow to decrease or even avoid these negative effects [32, 33]. One such recently proposed therapeutic target in the treatment of autoimmune diseases is the FAN (Factor Associated with Neutral Sphingomyelinase Activation) protein encoded by the NSMAF gene [34]. FAN is an adaptor molecule, which, through its interaction with the NSD domain of the TNF-R1 receptor, modulates expression of genes induced by the TNF-a; namely, genes encoding inflammatory proteins, such as interleukin 6 (IL-6), and chemokines, e.g., CCL5, CCL9, CCL20 and CXCL-2 [34-36]. Research performed on mice showed that in FAN^{-/-} individuals, the TNF-α-induced expression of the aforementioned pro-inflammatory molecules was selectively altered. This resulted in a disruption of the leukocyte population in secondary lymphoid organs, as observed through the reduction of macrophages, neutrophils, dendritic and lymphoid cells number in the spleen [34, 36].

Aside from all drug treatments, there are also many known household methods of dealing with inflammation and immune responses [37-39]. One of the most effective and oldest methods is the use of cabbage (*Brassica oleracea* var. *capitata*), and more precisely applying cabbage leaves compresses or drinking the cabbage juice. Due to its specific properties, cabbage has been used in natural medicine mainly for rheumatic pain, veins and lymphatic vessels inflammation, bruises, sprains, mastitis, or gastrointestinal problems. Its "spectrum" of use is however much wider and encompasses treatment of both internal and external diseases [40-42]. As is suggested by the newest research, the effect of consumed plants on the human organism may be attributed not only to the minerals, antioxidants, vitamins or polypeptides contained therein, but also to the presence of small RNA molecules - miRNAs. The study by Zhang et al. provided evidence not only that food-derived miRNAs are abundant in human serum but also that they can negatively regulate expression of specific genes in mammals. For example, MIR168a inhibits expression of the low-density lipoprotein receptor adapter protein 1 (LDLRAP1) in liver and thereby disrupts LDL plasma homeostasis [43]. Plant-derived miRNAs were also identified by Wang et al., who showed that miRNA molecules compose a significant sRNAs fraction in human plasma [44]. These recent reports on the cross-kingdom regulation by plant miRNAs open a new door for natural medicine and the treatment of many known diseases.

In the patent application CN102887948 (publ. 2013-01-23) antibacterial and immunoregulation polypeptide medicine is described. The invention belongs to a biotechnology, and relates to an antibacterial and immunoregulation apoE-mimetic peptide medicine. The apoE-mimetic peptide ApoE23 has a structure represented by the sequence 1. *In vivo* and *in vitro* tests demonstrate that the apoE-mimetic peptide can lower the LPS-induced TNF-alpha, IL-6 and IL-10 expression in THP-1 cells and human peripheral blood mononuclear cells, obviously reduce the death rate of mice with septicopyemia, reduce the TNF-α, IL-6 and LPS concentrations in the blood plasma of the mice with septicopyemia, relieve the inflammation in the lung, liver, small intestine and spleen of the mice with septicopyemia, and can kill *Escherichia coli, Pseudomonas aeruginosa,* pan-drug resistant *Acinetobacter baumannii* and *Staphylococcus aureus.* The apoE-mimetic peptide can be used to prepare Gram-negative bacilli resistant and pan-drug resistant Gram-negative bacilli resistant polypeptide medicines and immunoregulation polypeptide medicines and anti-septicopyemia medicines.

Patent application WO2012153854 (publ. 2012-11-15) provides novel means for modulating the expression of cytokine-chemokine genes associated with inflammation and infection are described. Specifically, the invention provides an antisense nucleotide that comprises the sequence complementary to the 3'UTR of the CCL2, CCL20, CX3CL1, IL-23A, CD69, NF-κB p65, TNF-α Fam89a, Grk5, phosopholipid scramblase 1, Runx1, semaphorin 4A, Steap4, lymphotoxin β Psmb10 or TLR2 gene induced by IL-1β and that is capable of modulating the expression of the gene; sense oligonucleotide that contains the sequence complementary to the endogenous antisense transcription product containing the sequence complementary to the 3'UTR of the gene and that is capable of modulating the expression of the gene; and an agent that prevents and/or treats inflammatory disease or infection and contains the sense or antisense nucleotide.

In the patent application AU2012201409 (publ. 2012-03-29) RNA interference is provided for inhibition of tumor necrosis factor α (TNF-α) by silencing TNF-α cell surface receptor TNF receptor-I (TNFRI) mRNA expression, or by silencing 5 TNF-α converting enzyme (TACE/ADAM17) mRNA expression. Silencing such TNF-α targets, in particular, is useful for treating patients having a TNF-α-related condition or a risk of developing a TNF-α-related condition such as the ocular conditions dry eye, allergic conjunctivitis, or ocular inflammation, or such as dermatitis, rhinitis, or asthma, for example.

Patent application WO2012115469 (publ. 2012-08-30) relates to an antiinflammatory functional food composition for oral administration, and more particularly, to a method for extracting, from grain and fruit, active ingredients which are effective in preventing inflammation and improving symptoms, and to an anti-inflammatory functional food composition comprising the extracts for oral administration. To this end, the anti-inflammatory functional food composition for oral administration according to the invention is prepared by obtaining extracts of *Morus alba* L. (mulberry), black turtle beans (black beans), and onions, and mixing said extracts at an appropriate ratio. The thus-prepared functional food composition may be used as a composition for anti-inflammatory analgesics, and has the effects of preventing and alleviating the symptoms of arthritis, as it exhibits, in an appropriate capacity range, superior antioxidant effects, inflammatory edema relieving effects, acute inflammation and chronic inflammation inhibitory effects, effects of inhibiting the expression of lipoxygenase, cyclooxygenase-2, inducible nitric oxide synthase (iNOS) and type I collagenase which are pro-inflammatory enzymes, and effects of inhibiting the production of tumor necrosis factor-alpha (TNF-α), interleukin 1-beta (IL-1β), interleukin 6 (IL-6) and prostaglandins, which are pro-inflammatory factors.

In a patent application KR101090177 (publ. 2011-12-06) an IL-32 antagonistic RNA aptamer is provided to suppress TNF-α expression and to develop a therapeutic agent for inflammatory diseases. An RNA aptamer has a sequence of sequence number 7 and binds to IL-32 as an antagonist. The RNA aptamer blocks the function of intrinsic IL-32. The RNA aptamer has high affinity with IL-32. The RNA aptamer is applied for developing a therapeutic agent for treating chronic inflammation such as rheumatic arthritis.

The patent application JP2003267880 (publ. 2003-09-25) describes composition for antiallergy and antiinflammation, which has excellent stability, cost, and is safe. The interleukin-4 production inhibitor comprises *Brassica oleracea acephala* and/or *Brassica orelacea acephala* extract. The composition for antiallergy and the composition for antiinflammation comprise the interleukin-4 production inhibitor and have excellent effect on allergies such as allergic rhinitis, pollinosis, etc. These compositions are orally administered or used as skin care preparations.

Patent application WO03070897 (publ. 2003-08-28) concerns methods and reagents useful in modulating TNF superfamily and TNF receptor superfamily gene expression in a variety of applications, including use in therapeutic, diagnostic, target validation, and genomic discovery applications. Specifically, the invention relates to small nucleic acid molecules, such as short interfering nucleic acid (siNA), short interfering RNA (siRNA), double-stranded RNA (dsRNA), micro-RNA (miRNA), and short hairpin RNA (shRNA) molecules capable of mediating RNA interference (RNAi) against TNF superfamily and TNF receptor superfamily gene expression and/or activity. The small nucleic acid molecules are useful in the treatment of septic shock, rheumatoid arthritis, HIV and AIDS, psoriasis, inflammatory or autoimmune disorders and any other disease or condition that responds to modulation of TNF and/or TNF receptor expression or activity.

In the patent application US2008279862 (publ. 2008-11-13) methods for treating an inflammatory or an immune condition are described. The invention relates to methods for treating an inflammatory or an immune condition with IL-1 inhibitors and an inhibitor of B cell or T cell activation. Methods for treating an inflammatory or an immune condition with TNF inhibitors and an inhibitor of B cell or T cell activation are described.

In the patent application US2012301484 (publ. 2012-11-29) novel regulatory T cell proteins and uses thereof are described. One protein, designated PD-L3, resembles members of the PD-L1 family, and co-stimulates αCD3 proliferation of T cells in vitro. A second, TNF-like, protein has also been identified as being upregulated upon αCD3/αGITR stimulation. This protein has been designated T^{reg}-sTNF. Proteins, antibodies, activated T cells and methods for using the same are disclosed. In particular methods of using these proteins and compounds, preferably antibodies, which bind or modulate (agonize or antagonize) the activity of these proteins, as immune modulators and for the treatment of cancer, autoimmune disease, allergy, infection and inflammatory conditions, e.g. multiple sclerosis is disclosed.

Patent application DE4006768 (publ. 1991-09-05) relates to the antirheumatic agent (I) obtained from the juice of cabbage leaves, which also contains olive oil to give the mixture the right consistency. Preferably it is in the form of a salve, which contains (in addition to the olive oil) an emulsifier and a preservative. A preferred ratio: 10-80 wt.% leaf extract/juice and 10-80 wt.% olive oil; ideally the salve contains a total of 5-80 (10-40) wt.% olive oil and emulsifier. Preferably the olive oil is cold-pressed. The antirheumatic agent (I) alleviates inflammation and pain associated with rheumatism. It can also be used in cases of lumbago, migraine, headache, tendon inflammation, dislocation and swelling. If necessary the salve can be wrapped in tissues and used overnight as a compress.

In the patent application PL397846 (publ. 2003-07-18) method of treating TNF-α-related diseases, comprising administering of TNF-α inhibitors, including TNF-α antibodies is described.

The goal of the present invention is to provide a novel method of treating various types of disorders and conditions, which decreases the inflammatory response of the organism by means of using plant-derived miR172 molecule or its synthetic equivalent. In the present invention, the plant-derived miR172 molecules are represented, respectively, by bol-miR172a or bol-miR172b molecules derived from *Brassica oleracea* var. *capitata* (cabbage). Previous reports concerning the use of cabbage in the treatment of various diseases and conditions did not associate its medicinal properties with the presence of miRNA molecules. For purposes of the present invention, the bioinformatics analysis of the results obtained by high-throughput sequencing of *Brassica oleracea* sRNAs was carried out, which confirmed the presence and determined the relative quantity of miRNA molecules in mature cabbage leaves. In the next step of carrying out the invention, the prediction of human target genes for identified cabbage miRNAs was performed.

The aforementioned bioinformatics target genes prediction for *B. oleracea* miRNAs revealed that bol-miR172a and bol-miR172b molecules, present in considerable quantities in cabbage leaves, may interact with mRNA encoding FAN protein and therefore negatively regulate its expression. The FAN protein mediates inflammatory responses by interacting with tumor necrosis factor receptor-1 (TNF-R1) and for this reason become a novel, potential therapeutic target in the treatment of autoimmune diseases [34, 36]. The carried out for the purposes of the present invention, measurements of FAN protein quantity performed on PBMCs (Peripheral Blood Mononuclear Cells) with the use of the ELISA assay confirmed that the presence of bol-miR172a or bol-miR172b reduces the level of FAN protein. Moreover, an *in vitro* test assessing the function of T and B lymphocytes under the stimulation of specific mitogens revealed that abundance of the aforementioned miRNAs molecules (at an appropriate concentration) decrease T and B lymphocytes proliferation. Phenotypic evaluation of PBMCs, also performed for purposes of this invention, additionally showed that the presence of bol-miR172a, as well as simultaneous presence of bol-miR172a and specific mitogen, slightly alters fraction of CD25+ cells in lymphocyte CD3+ population.

Despite the existing prior art and the knowledge related to miRNAs, the research performed so far neither furnish any proofs supporting the influence of plant miRNA molecules on the regulation of inflammatory processes in the human organism nor provide methods of utilising said molecules for therapeutic purposes.

Realisation of the goal so specified, as well as showing of proofs that specific miRNA molecules are responsible for certain medical properties of some plants, have been achieved in the present invention.

The subject matter of the invention is the plant-derived miRNA molecule or its synthetic equivalent, selected from amongst miR172a or miR172b, wherein miR172a is represented by the sequence SEQ. ID No. 1 and miR172b by the sequence SEQ. ID No. 2, and wherein said molecule has at least 6 out of 7 nucleotides present in the "seed" region represented by the sequence GAAUCUU and has at least 75% sequence similarity to SEQ. ID No. 1 or SEQ. ID No. 2, for use in decreasing the inflammatory response or for preventing an increase in the inflammatory response of the organism.

Preferably, the plant-derived miRNA molecule or its synthetic equivalent is for reducing proliferation of T and B lymphocytes.

Preferably, the plant-derived miRNA molecule or its synthetic equivalent is for reducing the level of FAN (Factor Associated with Neutral Sphingomyelinase Activation) protein.

Preferably, the plant-derived miRNA molecule or its synthetic equivalent interacts with mRNA encoding FAN protein negatively regulating its expression and therefore reducing proliferation of T and B lymphocytes.

Preferably, the plant-derived miRNA molecule or its synthetic equivalent interacts with mRNA encoding FAN protein negatively regulating its expression and therefore reducing the level of the FAN protein.

Preferably, the expression of FAN protein is negatively regulated and the level of FAN protein is reduced.

Preferably, the plant-derived miRNA molecule or its synthetic equivalent is 6, 7, 8, 9, 10, 11, 12, 13, 14, 15, 16, 17, 18, 19, 20, 21, 22, 23, 24, 25, 26, 27 or 28 nucleotides long.

Preferably, the plant-derived miRNA molecule or its synthetic equivalent has 2'- or 3'-O-methylation of the ribose of last nucleotide at the 3' end.

Preferably, the inflammatory response of the organism is decreased by reducing proliferation of T and B lymphocytes.

Preferably, the inflammatory response of the organism is decreased by reducing the level of FAN protein.

The attached Figures allow for a better understanding of the essence of the present invention.

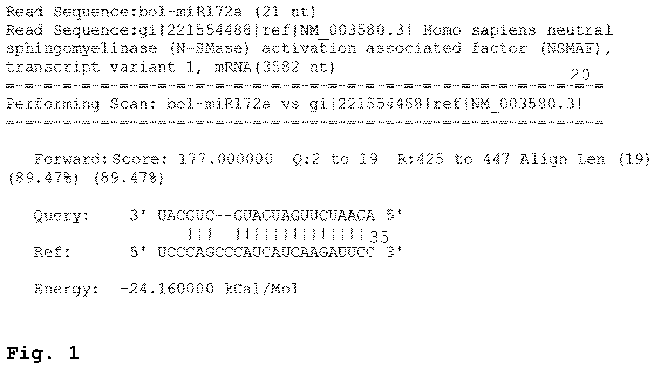

**Figure 1** The binding site of mRNA encoding FAN protein (marked as "Reference") predicted for bol-miR172a molecule (marked as "Query"). In the alignment "|" refers to a perfect complementarity between bases, "-" represents a gap, while ":" stands for a G:U wobble pair. The calculated score for presented alignment is 177, while minimum free energy of the structure (MFE) was -24.16 kcal/mol.

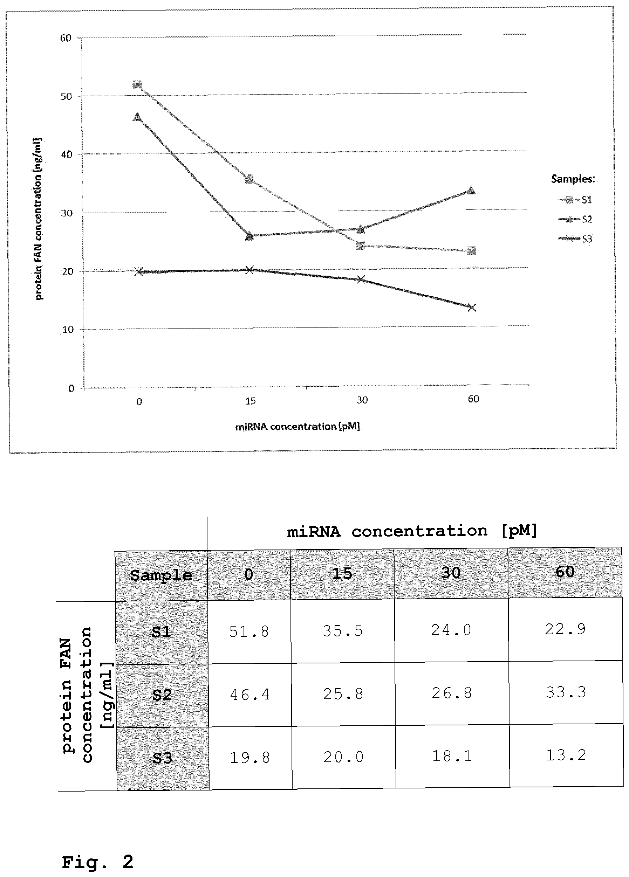

**Figure 2** Results of the measurements of FAN protein concentration (ng/ml) depending on the bol-miR172a molecule (presence at 3 different concentrations). The *in vitro* ELISA assay was performed on human peripheral blood MNCs. The presented results are mean values (ng/ml) calculated from two replicates.

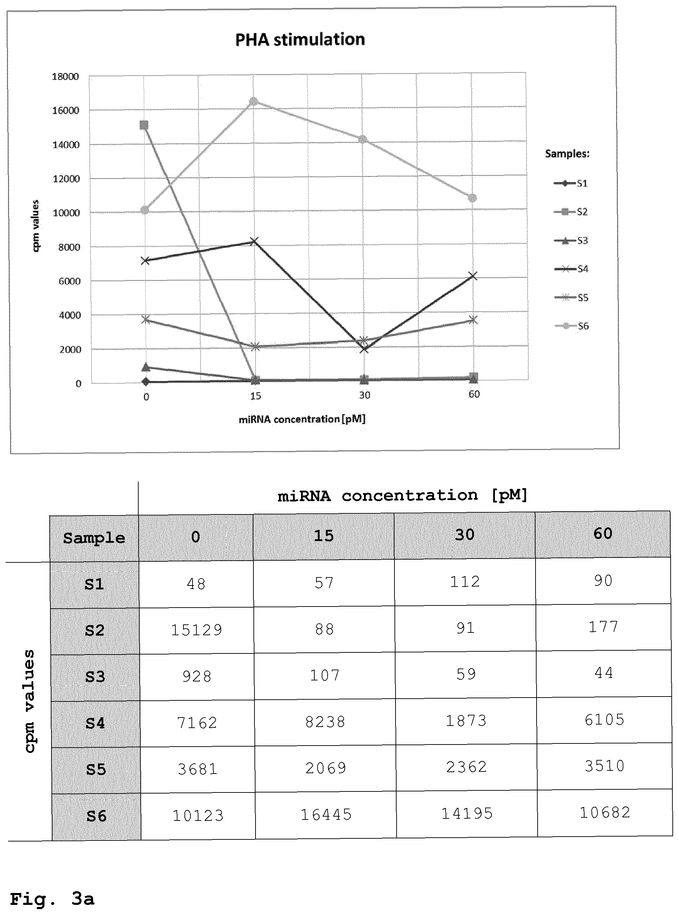

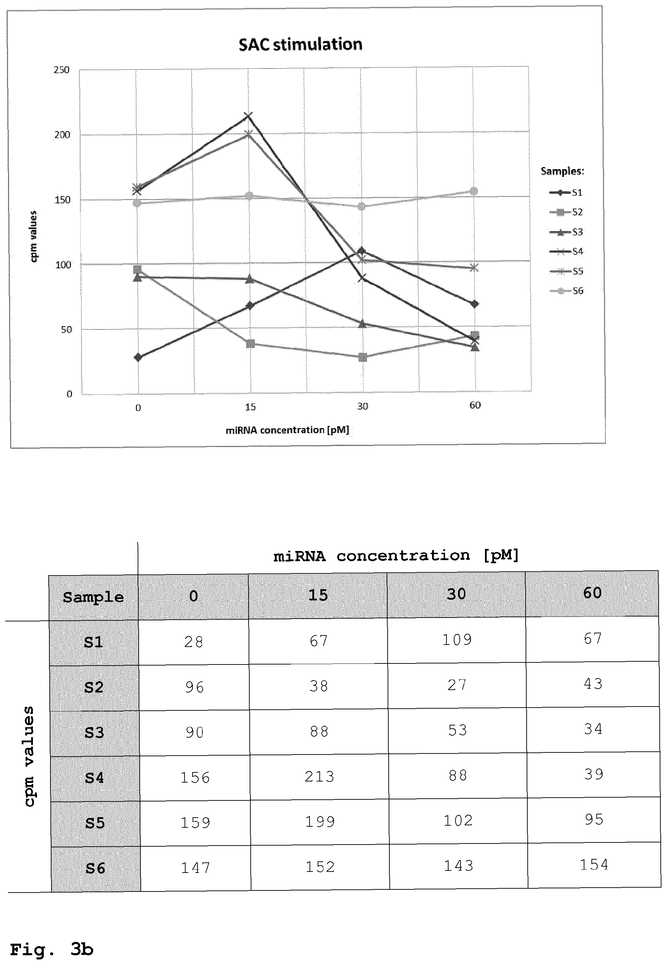

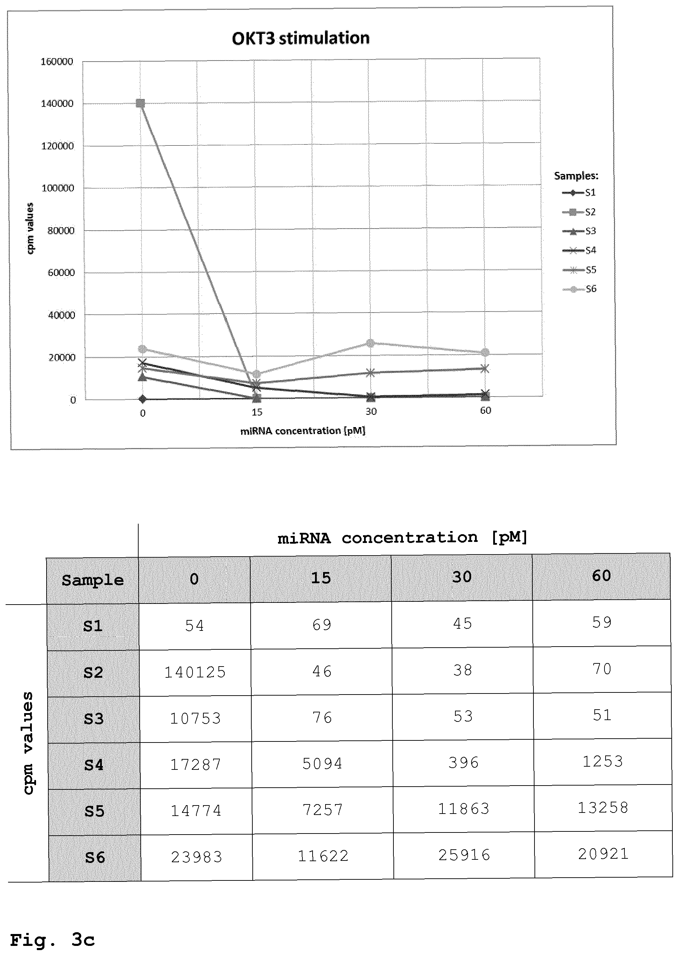

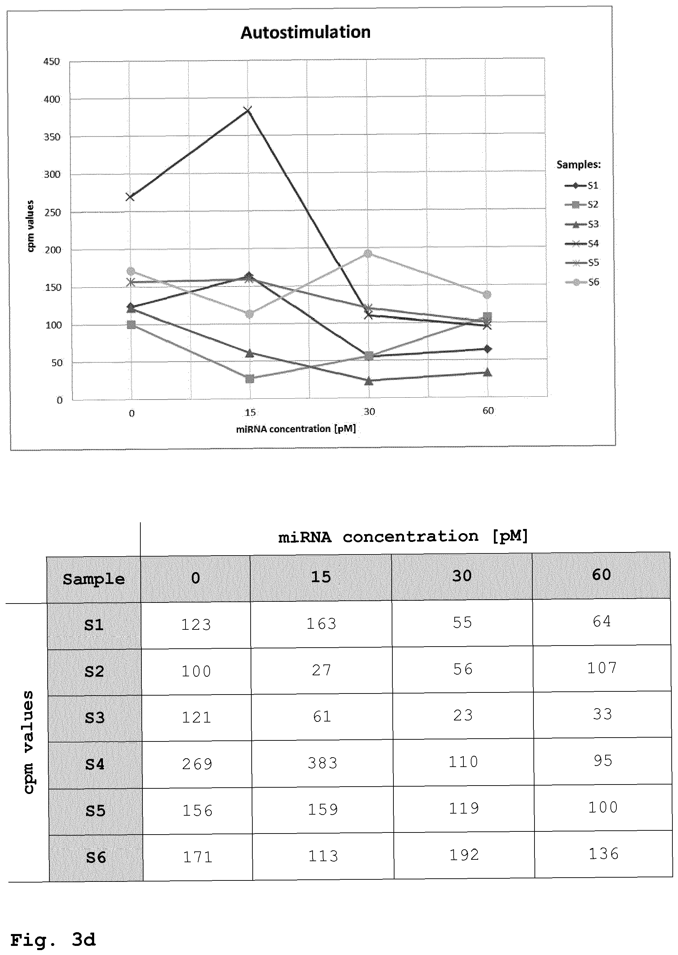

**Figure 3** Results of the *in vitro* assay for the human T and B lymphocyte function, where Fig. 3a shows the influence of the bol-miR172a molecule (present at 3 different concentrations) on the proliferation of T lymphocytes stimulated by phytohemagglutinin (PHA), Fig. 3b shows the influence of the bol-miR172a (present at 3 different concentrations) on the proliferation of B lymphocytes stimulated by *Staphylococcus aureus* (Cowan strain, SAC), Fig. 3c shows the influence of the bol-miR172a (present at 3 different concentrations) on the proliferation of T lymphocytes stimulated by anti-CD3 antibodies (OKT3), and Fig. 3d shows the influence of the bol-miR172a (present at 3 different concentrations) on the proliferation of B lymphocytes in the case of their auto stimulation. The presented results are mean cpm values calculated from three replicates.

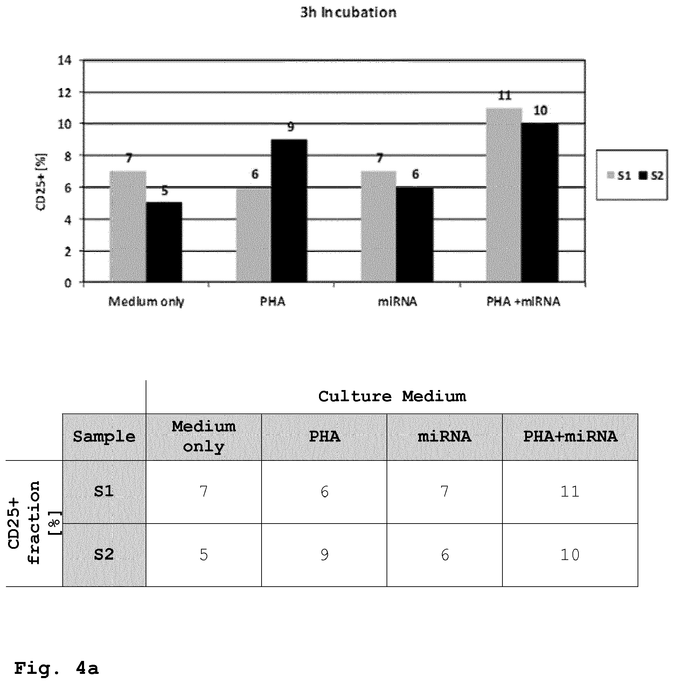

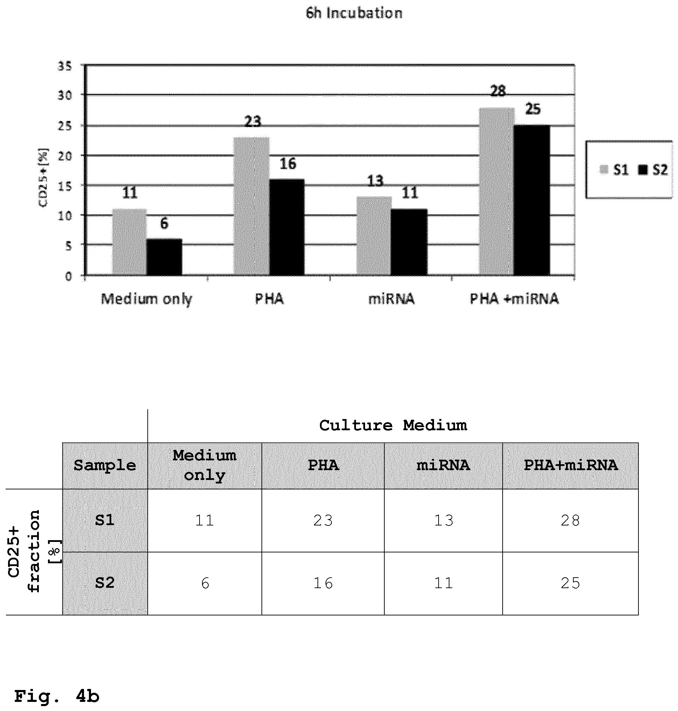

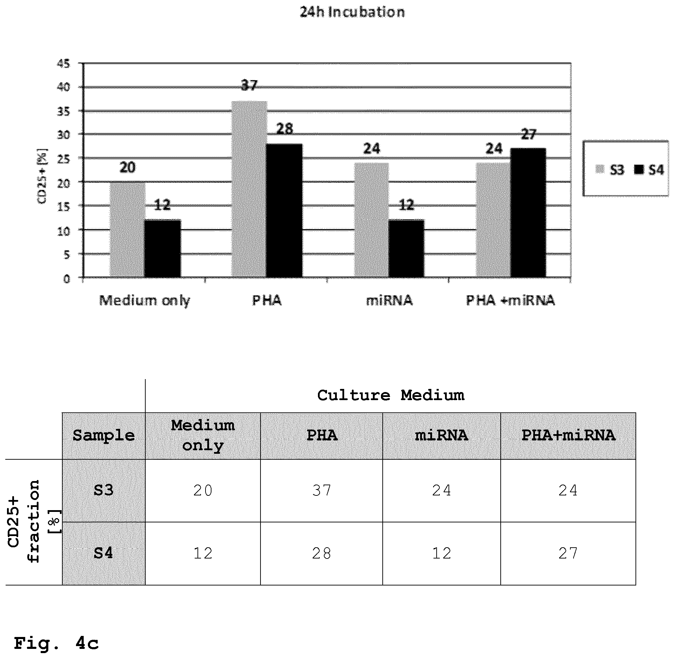

**Figure 4** Results of the phenotypic evaluation of human PBMCs using flow cytometry, where Fig. 4a shows the results of phenotypic evaluation of PMNCs incubated with miRNA and miRNA + PHA mitogen for 3 hours, Fig. 4b shows the results of phenotypic evaluation of PMNCs incubated with miRNA and miRNA + PHA mitogen for 6 hours, and Fig. 4c shows the results of phenotypic evaluation of PMNCs incubated with miRNA and miRNA + PHA mitogen for 24 hours. The presented results constitute the fraction (%) of CD25+ cells in lymphocyte CD3+ population are mean values calculated from three replicates.

Herein below are shown exemplary modes of carrying out the invention defined above.

### EXAMPLES

### MATERIAL AND METHODS

### Example 1

### High-throughput Sequencing Identification of miRNA Molecules from the Mature Brassica oleracea Leaves

Using the modified Trizol method as described by Szarzynska et al. [45], total RNA enriched with sRNA was isolated from the mature cabbage leaves (*Brassica oleracea* var. capitata, cultivar Balbro) in three independent biological replicates. Next generation sequencing (NGS) using the Illumina HiSeq technology was performed according to the manufacturer's protocol. Sequencing reads were generated from three constructed small RNA libraries. Each raw sRNAs dataset was further bioinformatically analyzed to clean, remove unnecessary tags and identify sequences representing conserved and novel miRNAs. The relative quantities of discovered cabbage miRNA molecules were estimated on the basis of mean values, calculated from the normalized number of miRNA tags in all three libraries [46]. The data obtained from small RNAs sequencing have been deposited in the NCBI's Gene Expression Omnibus repository under accession number GSE45578.

### Example 2

### Prediction of Putative Human Target Genes for Brassica oleracea miRNAs

The sequences of the identified conserved and novel cabbage miRNAs were used to predict their potential human target genes. The *Homo sapiens* 3'UTR, 5'UTR and CDS sequences, that were downloaded from the UCSC Genome Bioinformatics Site (http://genome.ucsc.edu/) and NCBI CCDS Database (www.ncbi.nlm.nih.gov/CCDS/CcdsBrowse.cgi) [47], respectively, served as reference target genes dataset. The bioinformatics targets prediction was performed using miRanda method (www.microrna.org/microrna/getDownloads.do). The miRanda search procedure examines sequence complementarity, interspecies conservation and thermodynamic stability of the miRNA:mRNA duplex [48]. The prediction parameters were as follows: (1) G:U base pairing was permitted but scored lower (score +2) than canonical base pairs (score +5), (2) the alignments with gaps and non-canonical base pairs in the "seed" region (2-8 nt at the 5' end of the molecule) were discarded, and (3) alignments with scores over 130 and minimum free energy (MFE) of the structure less than -17 kcal/mol were selected. The generated list of putative human targets genes was sorted by the highest alignment score and lowest MFE of the structure. To designate potential processes involving the predicted mRNA sequences and to suggest a probable influence of cabbage miRNAs on human organism, the selected targets were mapped on the UniProt database (www.uniprot.org) and annotated using the Blast2GO (http://www.blast2go.com/b2ghome) software [49]. The obtained dataset was analysed manually and the most interesting, for the invention, human target gene for cabbage bol-miR172 was selected, namely mRNA encoding FAN protein represented by the sequence SEQ. ID No. 3.

### Example 3

### Measurement of FAN Protein Quantity Using ELISA Assay

Mononuclear cells (MNCs) were isolated from peripheral blood of healthy donors by density gradient centrifugation for 12 min (2500 rpm, room temperature) using Ficoll- Histopaque (1077 g/l). Isolated MNCs were washed twice with 0.9% NaCl solution (1800 rpm, 10 min) and cultured in Parker medium supplemented with L-glutamine (2 mM), 3-β-mercaptoethanol, HEPES (0.23%), fetal calf serum (FCS, 10%) and gentamicin (0.1 mg/ml). The appropriate volume of miRNA was added to the cultures to yield a 15 pM, 30 pM and 60 pM concentrations. LPS in a 1 µg/ml concentration (Sigma-Aldrich) was used to stimulate cells. After addition of LPS, cells were incubated at 37°C, in 5% CO₂ humidified atmosphere for 72 h. Next, cells were collected, centrifuged (1800 rpm, 10 min) and washed twice with phosphate-buffered saline (PBS). Centrifuged cells were resuspended in 200 µl of PBS and frozen in -20°C. According to the ELISA kit manufacturer's protocol (Cloud-Clone Corp.), cells were frozen and thawed three times before FAN protein labelling. The level of FAN protein was determined in the collected supernatant using the LEDETECT96 plate reader at 450 nm wavelength.

### Example 4

### In vitro Proliferation Assay for Lymphocyte Function

### Peripheral Blood Mononuclear Cells (PBMCs) Isolation:

Mononuclear cells (MNCs) were isolated from peripheral blood of healthy donors by gradient centrifugation using Gradisol (Ficoll) (1077 g/l). Next, they were counted in a Bürker chamber using Türk solution. Isolated MNCs were diluted to 1x10⁶ cells/ml concentration in culture medium. The cell lines were cultured in Parker medium supplemented with L-glutamine (2 mM), 3-β-mercaptoethanol, HEPES (0.23%), fetal calf serum (FCS, 10%) and gentamicin (0.1 mg/ml).

### Proliferation Test :

The lymphocytes cultures were seeded into a 96-well plate with 1x10⁵ cells/well and stimulated by specific mitogens: anti-CD3 monoclonal antibodies (OKT3, 1 µg/ml), phytohemagglutinin (PHA, 20 µg/ml, Sigma-Aldrich) and *Staphylococcus aureus* Cowan strain (SAC, 0.004%) suspension. To each well the appropriate volume of miRNA was added to yield a 15 pM, 30 pM and 60 pM concentrations. The control cultures contained the equivalent volume of culture medium only. The prepared cultures were incubated at 37°C, in 5% CO₂ humidified atmosphere for 72 h. After addition of the [³H] thymidine, incubation was continued for 17 h in the aforementioned conditions. The cell cultures with [³H] thymidine incorporated into DNA, were transferred from plate onto filter paper by automatic harvester. For cell counting the scintillation counter was used. The results are reported as counts per minute (cpm).

### Example 5

### Evaluation of PBMCs Phenotype under PHA Mitogen Stimulation and miRNA Abundance

The evaluation of CD25+ (a chain of IL-2 receptor) cells subpopulation in MNCs population was performed with the use of immunofluorescence technique and flow cytometry. Mononuclear cells (MNCs) were isolated from peripheral blood of healthy donors by gradient centrifugation using Gradisol L (1077 g/l). Next, the isolated MNCs (in 1 x 10⁶ cells/ml concentration) were incubated for 3 h, 6 h and 24 h with miRNA in 30 pM concentration, after PHA (Sigma-Aldrich) stimulation in 20 µg/ml and 30 µg/ml concentration. Cell's phenotype was labelled with the appropriate monoclonal antibodies CD25-FITC and CD3-PE (Becton-Dickinson) by bicolor immunofluorescence technique. The MNCs were incubated with the antibodies for 15 min in room temperature, without access to the light. The labeled cells were then washed twice with PBS supplemented with 0.1% FCS. Next, they were resuspended in 500 µl of FACSFlow and detected by the flow cytometer F ACSCalibur (Becton-Dickinson). Data acquisition, storing and analysis was performed with the use of CellQuest software (Becton-Dickinson). The results were presented as CD25+ cells fraction in CD3+ cells (lymphocytes T) population.

### RESULTS

### Identification of miRNA Molecules in Mature Cabbage Leaves

To identify a larger set of miRNA molecules in the mature *Brassica oleracea* var. *capitata* leaves the high-throughput sequencing of three independent sRNA libraries was performed. The generated raw reads were further bioinformatically processed. As a result of aforementioned analysis, 261 conserved miRNAs (belonging to 62 families) were discovered (together with their estimated quantities) [46]. Among the most abundant molecules, the bol-miR172a (SEQ. ID No. 1) and bol-miR172b (SEQ. ID No. 2), which are the subject matter of the invention, were found.

### Potential Human Target Genes Prediction for Identified Brassica oleracea miRNAs

In the next step of carrying out the invention, prediction of putative human target genes for cabbage miRNAs molecules was performed. The results generated by miRanda method were sorted by the highest alignment score and lowest MFE of the structure. Among the predicted human targets the most interesting for the invention and associated with the inflammatory processes, was the mRNA molecule encoding FAN protein (SEQ. ID No. 3). Molecules bol-miR172a (SEQ. ID No. 1) and bol-miR172b (SEQ. ID No. 2), which are present in a large quantities in cabbage leaves, were selected to interact with the mRNA encoding FAN protein. The aforementioned hybridization occurs, according to the method used, in the CDS region of the target mRNA. The sequence complementarity of the miRNA and mRNA binding site was designated to 89.4%. The calculated alignment score was 177, while minimum free energy of the structure was -24.16 kcal/mol (Fig. 1).

### Influence of miRNA Abundance on FAN Protein Level

An important step in the implementation of the invention was to determine the impact of the molecule bol-miR172a or bol-miR172b on the FAN protein level. Performed on the human PBMCs, *in vitro* analysis with the use of ELISA method showed that abundance of bol-miR172a in a concentration of 15 pM, 30 pM, as well as 60 pM reduces significantly level of the FAN protein in analyzed preparation. The greatest decrease of FAN protein quantity was observed in a presence of bol-miR172a in 30 pM concentration (Fig. 2).

### Effect of miRNA Abundance and Mitogen Stimulation on T and B Lymphocyte Proliferation

*In vitro* evaluation of the human T and B lymphocyte function associated with their autostimulation or stimulation by specific mitogen, such as PHA, SAC and OKT3, showed that abundance of bol-miR172a in 30 pM concentration significantly reduce the B and T lymphocyte proliferation in the case of their autostimulation or presence of a specific mitogen (Fig. 3a, b, c and d).

### Phenotypic Evaluation of MNCs by Flow Cytometry

Flow cytometric analysis of human PBMCs incubated for 3 h, 6 h and 24 h with bol-miR172a molecule (in 30 pM concentration), confirmed the slightly altered fraction of CD25+ cells in lymphocyte CD3+ population relative to the control.
Additionally, similar minor changes in fraction of CD25+ cells in lymphocyte CD3+ population relative to the control were observed in the case of the simultaneous presence of bol-miR172a molecule (in 30 pM concentration) and PHA mitogen in 30 µg/ml concentration (Fig. 4a, b and c).

### To Summarize:

Performed *in vitro* lymphocyte proliferation assay, which measures lymphocytes ability to proliferate in response to various stimuli, showed that presence of bol-miR172a molecule reduces the proliferation of B lymphocytes, as well as T lymphocytes in the case of their autostimulation or stimulation by SAC, OKT3 and PHA mitogen, respectively. Moreover, the phenotype evaluation of PBMCs using flow cytometry method revealed that abundance of the bol-miR172a molecule, with or without the simultaneous presence of the PHA mitogen, slightly changes the fraction of the CD25+ cells in T cell population. Aforementioned results showed that bol-miR172a and bol-miR172b molecules are able to modulate B and T lymphocyte proliferation without affecting the activated T cells (CD25+) subpopulation. The mechanism responsible for the presented actions of bol-miR172a and bol-miR172b molecules is related to their interaction with mRNA encoding FAN protein. The influence of bol-miR172a molecule on the FAN protein level in PBMCs was evaluated by *in vitro* ELISA assay. Performed analysis revealed that abundance of the bol-miR172a (in all tested concentrations) results in a significant reduction of the FAN protein level.

### SEQUENCE LISTING

**SEQ. ID No. 1**
   **miR172a, RNA molecule, 21 nt**
   AGAAUCUUGAUGAUGCUGCAU
**SEQ. ID No. 2**
   **miR172b, RNA molecule, 21nt**
   AGAAUCUUGAUGAUGCUGCAU
**SEQ. ID No. 3**
   **NM_003580.3, *Homo sapiens* neutral sphingomyelinase (N-SMase) activation associated factor (NSMAF), transcript variant 1, mRNA, 3582 bp**

GCCGGAGUCCCACGCGAGGAUGCUGCGGUGAGCGCGGCCGGGACGCCGCGUCGGGCUCUCG
GCCGCCCAGCGGCGCCGCAGGGGAAGCCAGGCCGGCAGCCGCGCUCCCAGCCGGCCACCA
AUCCCGCCCUCCCCGGCUCCUCCGGACCUCCUCGGCAGGCGGCGGCGGGGCCUGCCUGUGCGC
UCUGCGCCCGCCCGCGCCUACCCUCCAUGGCGUUUAUCCGGAAGAAGCAGCAGGAGCAGCAGC
UGCAGCUCUACUCCAAGGAGAGAUUUCCUUGCUGCUGCUUAACUUGGAGGAGUACUACUUU
GAACAGCAUAGAGCCAAUCACAUUUUGCACAAGGGCAGUCACCAUGAAAGGAAAAUCAGAGG
CUCCUUAAAAAUAUGUUCAAAAUCGGUGAUUUUUGAACCAGAUUCAAUAUCCCAGCCCAUCA
UCAAGAUUCCUUUGAGAGACUGUAUAAAAAUAGGAAAGCAUGGAGAAAAUGGAGCCAAUAG
ACACUUCACAAAGGCAAAAUCUGGGGGUAUUUCACUCAUUUUCAGUCAGGUAUAUUUCAUUA
AAGAACAUAAUGUUGUUGCACCAUAUAAAAUAGAAAGGGGCAAAAUGGAAUAUGUUUUUGA
AUUGGAUGUUCCCGGGAAAGUGGAAGAUGUUGUGGAGACGUUGCUUCAGCUUCACAGAGCA
UCCUGCCUUGACAAAUUGGGUGACCAAACCGCCAUGAUAACAGCUAUUUGCAGUCUCGUUU
AGCUAGAACAUCAUUUGACAAAAACAGGUUCCAAAACAUUUCUGAAAAGCUGCACAUGGAAU
GCAAAGCAGAAAUGGUGACGCCUCUGGUGACUAAUCCUGGACACGUGUGCAUCACGGACACA
AACCUGUAUUUCAGCCCCUCAACGGCUACCCGAAACCUGUGGUCCAGAUAACACUCCAAGA
UGUCCGCCGCAUCUACAAAAGGAGGCACGGCCUCAUGCCUCUGGGCUUGGAAGUAUUUGCA
CAGAAGAUGAUCUGUGUUCCGACAUCUACCUAAAGUUCUAUGAACCUCAAGAUAGAGAUGAU
CUCUAUUUUACAUUGCCACAUACCUAGAGCACCAUGUGGCGGAGCACACUGCUGAGAGCUA
CAUGCUGCAGUGGCAGCGUGGACACCUUUCCAACUAUCAGUACCUCCUUCACCUCAACAACC
UGGCCGACCGCAGCUGCAACGACCUCUCCCAGUACCCUGUGUUCCAUGGAUAAUACAUGAU
UAUUCCAGCUCAGAACUAGAUUUGUCAAAUCCAGGAACCUUCCGGGAUCUCAGUAAGCCAGU
AGGGGCCCUAAAUAAGGAACGGCUGGAGAGACUACUGACACGCUACCAGGAAAUGCCUGAAC
CAAAGUUCAUGUAUGGGAGUCACUACUCUUCCCCGGGUUAUGUACUUUUUUAUCUUGUUAGG
AUUGCACCAGAGUAUAUGCUGUGCCUGCAGAAUGGAAGAUUUGAUAAUGCAGAUAGAAUGU
UCAACAGUAUUGCAGAAACUUGGAAAAACUGUCUGGAUGGUGCAACGGAUUUUAAAGAGUU
AAUUCCAGAAUUCUAUGGUGAUGAUGUGAGCUUUCUAGUCAAUAGCCUGAAGUUGGAUUUG
GGAAAGAGACAAGGAGGACAGAUGGUUGACGACGUGGAGCUUCCCCCUUGGGCUUCCAGUCC
CGAGGACUUUCUCCAGAAGAGCAAAGAUGCAUUGGAAAGCAAUUAUGUGUCUGAACACCUUC
ACGAGUGGAUUGAUCUAAUAUUUGGCUACAAACAAAAAGGGAGUGAUGCAGUUGGGGCCCA
UAAUGUAUUUCAUCCCCUGACCUAUGAAGGAGGUGUAGACUUGAACAGCAUCCAGGAUCCUG
AUGAGAAGGUAGCCAUGCUUACGCAAAUCUUGGAAUUUGGGCAGACACCAAAACAACUAUUU
GUGACCACCACAUCCUCGAAGGAUCACCCCAAAGUUAAAGUUGUCCCAGACCUCCAGUUA
UAAUGCUUCUAUGGCAGAUUCCCCAGGUGAAGAGUCUUUUGAAGACCUGACCGAAGAAAGCA

AAACACUGGCCUGGAAUAACAUCACCAAACUGCAGUUACACGAGCACUAUAAAAUCCACAAA
GAAGCAGUUACUGGAAUCACGGUCUCUCGCAAUGGAUCUUCAGUAUUCACAACAUCCCAAGA
UUCCACCUUGAAGAUGUUUUCUAAAGAAUCAAAAAUGCUACAAAGAAGUAUAUCAUUUUCA
AAUAUGGCUUUAUCGUCUUGUUUACUUUUACCAGGAGAUGCCACUGUCAUAACUUCUUCAUG
GGAUAAUAAUGUCUAUUUUUAUUCCAUAGCAUUUGGAAGACGCCAGGACACGUUAAUGGGA
CAUGAUGAUGCUGUUAGUAAGAUCUGUUGGCAUGACAACAGGCUAUAUUCUGCAUCGUGGG
ACUCUACAGUGAAGGUGUGGUCUGGUGUUCCUGCAGAGAUGCCAGGCACCAAAAGACACCAC
UUUGACUUGCUGGCCGAGCUGGAACAUGAUGUCAGUGUAGAUACAAUCAGUUUAAAUGCUGC
AAGCACACUGUUAGUUUCCGGCACCAAAGAAGGCACAGUGAAUAUUUGGGACCUCACAACGG
CCACCUUAAUGCACCAGAUUCCAUGCCAUUCAGGGAUUGUAUGUGACACUGCUUUUAGCCCA
GAUAGUCGCCAUGUCCUCAGCACAGGAACAGAUGGCUGUCUUAAUGUCAUUGAUGUGCAGAC
AGGAAUGCUCAUCUCCUCCAUGACAUCAGAUGAGCCCCAGAGGUGCUUUGUCUGGGAUGGAA
AUUCCGUUUUAUCUGGCAGUCAGUCUGGUGAACUGCUCGUUUGGGACCUCCUUGGAGCAAAA
AUCAGUGAGAGAAUACAGGGCCACACAGGUGCUGUGACAUGUAUAUGGAUGAAUGAACAGU
GUAGCAGUAUCAUCACAGGAGGGGAAGACAGACAAAUUAUAUUCUGGAAAUUGCAGUAUUA
AGUGCCUUUUCCUCUCCUGAAUAUUAAAUUGAACUCUAUUUAAUGCAUUUUUAAACCAAACU
UUUAAACGGACUGGUGAAUGUGCAAUGUUAGUAAUUAGAAGUUUUACCACAUGGAAAAUUU
GUGGUUUUAAACUUUCUAAAUCAUGGUGACUUCAUUGAAAGCCAUUAGUUGCUAUUCUCUU
AGGGCAGAUAAAAUGCGGCUGUGUUAGGAAAAACAUGUUACACUGUAAGGCAGAUGAUCGU
CCCCGUAUGAUGAUUGUCAGAAGACAGGACUAAGUAGCAGAGAAUAGCUAAGAGAUAAAUU
GGGCUGGGGAAACUUGUCAGAAAGCACUGAACAAUUAAGAAAUUUUCCAAGAAAAUGUGCA
GUAUUCUCUGCUACUUCUGAAUCUGUUUUGUCUUCCUAAUCUAUCACAAUUGCCACCCAUCG
GGUUUUGGGUGUGUGUUUUCAUAGCGUGGUUACUUUCUAUAAUGCUGUACCCAGAUUCUAA
GAACCUGGAGAAGGAUUAGCAGUUCUUAGUAAGUUUACUGUGUAUAGGAACGGUUUGUAUU
UCAUUACAGCUAUUCAUCUUUUCUACAUUAAAAAUAUUUUUCUCUAAAGAAAAAAAAAAAA
AAA

### REFERENCES:

1. Liu N, Okamura K, Tyler DM, Phillips MD, Chung WJ, Lai EC: The evolution and functional diversification of animal microRNA genes. Cell research 2008, 18(10):985-996.
2. Jones-Rhoades MW: Conservation and divergence in plant microRNAs. Plant molecular biology 2012, 80(1):3-16.
3. Niwa R, Slack FJ: The evolution of animal microRNA function. Current opinion in genetics & development 2007, 17(2):145-150.
4. Djuranovic S, Nahvi A, Green R: miRNA-mediated gene silencing by translational repression followed by mRNA deadenylation and decay. Science 2012, 336(6078):237-240.
5. Hu W, Coller J: What comes first: translational repression or mRNA degradation? The deepening mystery of microRNA function. Cell research 2012, 22(9):1322-1324.
6. Ying SY, Chang DC, Lin SL: The microRNA (miRNA): overview of the RNA genes that modulate gene function. Molecular biotechnology 2008, 38(3):257-268.
7. Yang Z, Ebright YW, Yu B, Chen X: HEN1 recognizes 21-24 nt small RNA duplexes and deposits a methyl group onto the 2' OH of the 3' terminal nucleotide. Nucleic acids research 2006, 34(2):667-675.
8. Bartel DP: MicroRNAs: genomics, biogenesis, mechanism, and function. Cell 2004, 116(2):281-297.
9. Chen X: MicroRNA biogenesis and function in plants. FEBS letters 2005, 579(26):5923-5931.
10. Kim VN, Han J, Siomi MC: Biogenesis of small RNAs in animals. Nature reviews Molecular cell biology 2009, 10(2):126-139.
11. Khraiwesh B, Arif MA, Seumel GI, Ossowski S, Weigel D, Reski R, Frank W: Transcriptional control of gene expression by microRNAs. Cell 2010, 140(1):111-122.
12. Kawamata T, Tomari Y: Making RISC· Trends in biochemical sciences 2010, 35(7):368-376.
13. Sontheimer EJ: Assembly and function of RNA silencing complexes. Nature reviews Molecular cell biology 2005, 6(2):127-138.
14. Zhang B, Wang Q, Pan X: MicroRNAs and their regulatory roles in animals and plants· Journal of cellular physiology 2007, 210(2):279-289.
15. Wienholds E, Plasterk RH: MicroRNA function in animal development. FEBS letters 2005, 579(26):5911-5922 .
16. Esteller M: Non-coding RNAs in human disease. Nature reviews Genetics 2011, 12(12):861-874.
17. De Guire V, Robitaille R, Tetreault N, Guerin R, Menard C, Bambace N, Sapieha P: Circulating miRNAs as sensitive and specific biomarkers for the diagnosis and monitoring of human diseases: promises and challenges. Clinical biochemistry 2013, 46(10-11):846-860.
18. Broderick JA, Zamore PD: MicroRNA therapeutics. Gene therapy 2011, 18(12):1104-1110.
19. Mack GS: MicroRNA gets down to business. Nature biotechnology 2007, 25(6):631-638.
20. Xiao C, Rajewsky K: MicroRNA control in the immune system: basic principles. Cell 2009, 136(1):26-36.
21. Hukowska-Szematowicz B, Tokarz-Deptula B, Deptula W: MicroRNA (miRNA) and the immune system. Centr Eur J Immunol 2012, 37(4):387-390.
22. Liu G, Abraham E: MicroRNAs in immune response and macrophage polarization. Arteriosclerosis, thrombosis, and vascular biology 2013, 33(2):170-177.
23. Filkova M, Jungel A, Gay RE, Gay S: MicroRNAs in rheumatoid arthritis: potential role in diagnosis and therapy. BioDrugs : clinical immunotherapeutics, biopharmaceuticals and gene therapy 2012, 26(3):131-141.
24. Boissier MC, Semerano L, Challal S, Saidenberg-Kermanac'h N, Falgarone G: Rheumatoid arthritis: from autoimmunity to synovitis and joint destruction. Journal of autoimmunity 2012, 39(3):222-228**.**
25. Simoni Y, Diana J, Ghazarian L, Beaudoin L, Lehuen A: Therapeutic manipulation of natural killer (NK) T cells in autoimmunity: are we close to reality? Clinical and experimental immunology 2013, 171(1):8-19.
26. Fournier C: Where do T cells stand in rheumatoid arthritis? Joint, bone, spine : revue du rhumatisme 2005, 72(6):527-532.
27. Rickert RC, Jellusova J, Miletic AV: Signaling by the tumor necrosis factor receptor superfamily in B-cell biology and disease. Immunological reviews 2011, 244(1):115-133.
28. Stinissen P, Raus J, Zhang J: Autoimmune pathogenesis of multiple sclerosis: role of autoreactive T lymphocytes and new immunotherapeutic strategies. Critical reviews in immunology 1997, 17(1):33-75.
29. Krueger JG, Bowcock A: Psoriasis pathophysiology: current concepts of pathogenesis. Annals of the rheumatic diseases 2005, 64 Suppl 2:ii30-36.
30. Campbell KA, Lipinski MJ, Doran AC, Skaflen MD, Fuster V, McNamara CA: Lymphocytes and the adventitial immune response in atherosclerosis. Circulation research 2012, 110(6):889-900.
31. Youinou P, Jamin C, Saraux A: B-cell: a logical target for treatment of rheumatoid arthritis. Clinical and experimental rheumatology 2007, 25(2):318-328.
32. Huseyin TE Ozer, Ozbalkan Z: Clinical Efficacy of TNF-α Inhibitors: An Update. International Journal of Clinical Rheumatology 2010, 5(1):101-115.
33. Croft M, Benedict CA, Ware CF: Clinical targeting of the TNF and TNFR superfamilies. Nature reviews Drug discovery 2013, 12(2):147-168.
34. Montfort A, Martin PG, Levade T, Benoist H, Segui B: FAN (factor associated with neutral sphingomyelinase activation), a moonlighting protein in TNF-R1 signaling. Journal of leukocyte biology 2010, 88(5):897-903.
35. Palin K, Bluthe RM, McCusker RH, Levade T, Moos F, Dantzer R, Kelley KW: The type 1 TNF receptor and its associated adapter protein, FAN, are required for TNFalpha-induced sickness behavior. Psychopharmacology 2009, 201(4):549-556.
36. Montfort A, de Badts B, Douin-Echinard V, Martin PG, Iacovoni J, Nevoit C, Therville N, Garcia V, Bertrand MA, Bessieres MH et al: FAN stimulates TNF(alpha)-induced gene expression, leukocyte recruitment, and humoral response. J Immunol 2009, 183(8):5369-5378.
37. Challem J: The Inflammation Syndrome: The Complete Nutritional Program to Prevent and Reverse Heart Disease, Arthritis, Diabetes, Allergies, and Asthma: John Wiley & Sons; 2003.
38. Pizzorno JEJ, Murray MT, Joiner-Bey H: The Clinician's Handbook of Natural Medicine: Elsevier Health Sciences; 2008.
39. Torkos S: The Canadian Encyclopedia of Natural Medicine: John Wiley & Son; 2012.
40. Carper J: Apteka zywnosci. Nowe i niezwykle odkrycia leczniczego dzialania zywnosci: Vesper; 2008.
41. Gornicka J: Apteka natury: poradnik zdrowia : ziololecznictwo, akupresura, masaz shiatsu: AWM; 1997.
42. Munns A: Cabbage leaves: cabbage leaves can help inflammation of any body part. BMJ 2003, 327(7412):451.
43. Zhang L, Hou D, Chen X, Li D, Zhu L, Zhang Y, Li J, Bian Z, Liang X, Cai X et al: Exogenous plant MIR168a specifically targets mammalian LDLRAP1: evidence of cross-kingdom regulation by microRNA. Cell Res 2012, 22(1):107-126.
44. Wang K, Li H, Yuan Y, Etheridge A, Zhou Y, Huang D, Wilmes P, Galas D: The complex exogenous RNA spectra in human plasma: an interface with human gut biota? PLoS One 2012, 7(12):e51009.
45. Szarzynska B, Sobkowiak L, Pant BD, Balazadeh S, Scheible WR, Mueller-Roeber B, Jarmolowski A, Szweykowska-Kulinska Z: Gene structures and processing of Arabidopsis thaliana HYL1-dependent pri-miRNAs. Nucleic acids research 2009, 37(9):3083-3093.
46. Lukasik A, Pietrykowska H, Paczek L, Szweykowska-Kulinska Z, Zielenkiewicz P: High-throughput sequencing identification of novel and conserved miRNAs in the Brassica oleracea leaves. BMC genomics 2013, 14:801.
47. Pruitt KD, Harrow J, Harte RA, Wallin C, Diekhans M, Maglott DR, Searle S, Farrell CM, Loveland JE, Ruef BJ et al: The consensus coding sequence (CCDS) project: Identifying a common protein-coding gene set for the human and mouse genomes. Genome research 2009, 19(7):1316-1323.
48. Enright AJ, John B, Gaul U, Tuschl T, Sander C, Marks DS: MicroRNA targets in Drosophila. Genome biology 2003, 5(1):R1.
49. Conesa A, Gotz S, Garcia-Gomez JM, Terol J, Talon M, Robles M: Blast2GO: a universal tool for annotation, visualization and analysis in functional genomics research. Bioinformatics 2005, 21(18):3674-3676.

## Claims

1. Plant-derived miRNA molecule or its synthetic equivalent, selected from amongst miR172a or miR172b, wherein miR172a is represented by the sequence SEQ. ID No. 1 and miR172b by the sequence SEQ. ID No. 2, and wherein said molecule has at least 6 out of 7 nucleotides present in the "seed" region represented by the sequence GAAUCUU and has at least 75% sequence similarity to SEQ. ID No. 1 or SEQ. ID No. 2, for use in decreasing the inflammatory response or for preventing an increase in the inflammatory response of the organism.

2. The plant-derived miRNA molecule or its synthetic equivalent for use according to claim 1 for reducing proliferation of T and B lymphocytes.

3. The plant-derived miRNA molecule or its synthetic equivalent for use according to claim 1 for reducing the level of FAN (Factor Associated with Neutral Sphingomyelinase Activation) protein.

4. The plant-derived miRNA molecule or its synthetic equivalent for use according to claim 1 wherein the plant-derived miRNA molecule or its synthetic equivalent interacts with mRNA encoding FAN protein negatively regulating its expression and therefore reducing proliferation of T and B lymphocytes.

5. The plant-derived miRNA molecule or its synthetic equivalent for use according to claim 1 wherein the plant-derived miRNA molecule or its synthetic equivalent interacts with mRNA encoding FAN protein negatively regulating its expression and therefore reducing the level of the FAN protein.

6. The plant-derived miRNA molecule or its synthetic equivalent for use according to claim 5 wherein the expression of FAN protein is negatively regulated and the level of FAN protein is reduced.

7. The plant-derived miRNA molecule or its synthetic equivalent for use according to claim 1 wherein the molecule is 6, 7, 8, 9, 10, 11, 12, 13, 14, 15, 16, 17, 18, 19, 20, 21, 22, 23, 24, 25, 26, 27 or 28 nucleotides long.

8. The plant-derived miRNA molecule or its synthetic equivalent for use according to claim 1 wherein the molecule has 2'- or 3'-O-methylation of the ribose of last nucleotide at the 3'end.

9. The plant-derived miRNA molecule or its synthetic equivalent for use according to claim 1 wherein the inflammatory response of the organism is decreased by reducing proliferation of T and B lymphocytes.

10. The plant-derived miRNA molecule or its synthetic equivalent for use according to claim 1 wherein the inflammatory response of the organism is decreased by reducing the level of FAN protein.

## Patentansprüche

1. Pflanzen-stammendes miRNA Molekül oder sein synthetisches Äquivalent, ausgewählt aus miR172a oder miR172b, wobei miR172a mit der Sequenz SEQ. ID No. 1 und miR172b mit der Sequenz SEQ. ID No. 2 dargestellt ist, und wobei dieses Molekül mindestens 6 von 7 Nukleotiden im Seed-Region dargestellt mit der Sequenz GAAUCUU aufweist und mindestens 75% Sequenzähnlichkeit zu SEQ. ID No. 1 oder SEQ. ID No. 2 hat, zur Verwendung in Verringerung der Entzündungsreaktion oder Verhinderung der Entzündungsreaktionsteigerung des Körpers.

2. Pflanzen-stammendes miRNA Molekül oder sein synthetisches Äquivalent zur Verwendung gemäß Anspruch 1, zur Reduktion der Proliferation von T und B Lymphozyten.

3. Pflanzen-stammendes miRNA Molekül oder sein synthetisches Äquivalent zur Verwendung gemäß Anspruch 1, zur Reduktion des FAN (Factor Associated with Neutral Sphingomyelinase Activation) Proteinespiegels.

4. Pflanzen-stammendes miRNA Molekül oder sein synthetisches Äquivalent zur Verwendung gemäß Anspruch 1, wobei das Pflanzen-stammendes miRNA Molekül oder sein synthetisches Äquivalent eine Wechselwirkung mit mRNA kodierende FAN Protein negativ regulierende seine Expression aufweist und somit die Proliferation von T und B Lymphozyten reduziert.

5. Pflanzen-stammendes miRNA Molekül oder sein synthetisches Äquivalent zur Verwendung gemäß Anspruch 1, wobei das Pflanzen-stammendes miRNA Molekül oder sein synthetisches Äquivalent eine Wechselwirkung mit mRNA kodierende FAN Protein negativ regulierende seine Expression aufweist und somit den Spiegel von FAN Protein reduziert.

6. Pflanzen-stammendes miRNA Molekül oder sein synthetisches Äquivalent zur Verwendung gemäß Anspruch 5, wobei die Expression von FAN Protein negativ reguliert und der Spiegel von FAN Protein reduziert wird.

7. Pflanzen-stammendes miRNA Molekül oder sein synthetisches Äquivalent zur Verwendung gemäß Anspruch 1, wobei das Molekül eine Länge von 6, 7, 8, 9, 10, 11, 12, 13, 14, 15, 16, 17, 18, 19, 20, 21, 22, 23, 24, 25, 26, 27 oder 28 Nukleotiden aufweist.

8. Pflanzen-stammendes miRNA Molekül oder sein synthetisches Äquivalent zur Verwendung gemäß Anspruch 1, wobei das Molekül eine 2'- oder 3'-O-Methylation der Ribose am letzten 3'-Nukleotidende aufweist.

9. Pflanzen-stammendes miRNA Molekül oder sein synthetisches Äquivalent zur Verwendung gemäß Anspruch 1, wobei die Entzündungsreaktion des Körpers durch die Reduktion der Proliferation der T und B Lymphozyten verringert wird.

10. Pflanzen-stammendes miRNA Molekül oder sein synthetisches Äquivalent zur Verwendung gemäß Anspruch 1, wobei die Entzündungsreaktion des Körpers durch die Reduktion des Spiegels von FAN Protein verringert wird.

## Revendications

1. Molécule miARN dérivée de plante ou son équivalent synthétique, choisie parmi miR172a ou miR172b, où miR172a est représentée par la séquence SEQ. ID N° 1 et miR172b par la séquence SEQ. ID N° 2, et où ladite molécule a au moins 6 parmi 7 nucléotides présents dans la région "semence" représentée par la séquence GAAUCUU et a une similarité de séquence d'au moins 75% avec SEQ. ID N° 1 ou SEQ. ID N° 2, destinée à diminuer la réponse inflammatoire ou à prévenir une augmentation de la réponse inflammatoire de l'organisme.

2. Molécule miARN dérivée de plante ou son équivalent synthétique pour une utilisation selon la revendication 1 pour réduire la prolifération des lymphocytes T et B.

3. Molécule miARN dérivée de plante ou son équivalent synthétique pour une utilisation selon la revendication 1 pour réduire le taux de protéine FAN (facteur associé à l'activation de la sphingomyélinase neutre).

4. Molécule miARN dérivée de plante ou son équivalent synthétique pour une utilisation selon la revendication 1 où la molécule miARN dérivée de plante ou son équivalent synthétique interagit avec l'ARNm codant pour la protéine FAN régulant négativement son expression et réduisant ainsi la prolifération des lymphocytes T et B.

5. Molécule miARN dérivée de plante ou son équivalent synthétique pour une utilisation selon la revendication 1 où la molécule miARN dérivée de plante ou son équivalent synthétique interagit avec l'ARNm codant pour la protéine FAN régulant négativement son expression et réduisant ainsi le niveau de la protéine FAN.

6. Molécule miARN dérivée de plante ou son équivalent synthétique pour une utilisation selon la revendication 1 où l'expression de la protéine FAN est régulée négativement et le niveau de la protéine FAN est réduit.

7. Molécule miARN dérivée de plante ou son équivalent synthétique pour une utilisation selon la revendication 1 où la longueur de la molécule est de 6, 7, 8, 9, 10, 11, 12, 13, 14, 15, 16, 17, 18, 19, 20, 21, 22, 23, 24, 25, 26, 27 ou 28 nucléotides.

8. Molécule miARN dérivée de plante ou son équivalent synthétique pour une utilisation selon la revendication 1 où la molécule a une 2'- ou 3'-O-méthylation du ribose du dernier nucléotide à l'extrémité 3'.

9. Molécule miARN dérivée de plante ou son équivalent synthétique pour une utilisation selon la revendication 1 où la réponse inflammatoire de l'organisme est diminuée par la réduction de la prolifération des lymphocytes T et B.

10. Molécule miARN dérivée de plante ou son équivalent synthétique pour une utilisation selon la revendication 1 où la réponse inflammatoire de l'organisme est diminuée par la réduction du niveau de la protéine FAN.
